# EUROPEAN PATENT APPLICATION

(11) **EP 1 230 927 A1**
(43) Date of publication of application: **14.08.2002**
(21) Application number: 01103203.4
(22) Date of filing: 12.02.2001
(51) Int. Cl.: A61K 35/78, A61P 31/02

(54) **Phyto-derivative disinfectant solution for veterinary use in the endo-mammary anti-mastitis treatment of milk-producing animals**

(71) Applicant: Allegrini S.p.a., 24050 Grassobbio, (Bergamo) (IT)
(72) Inventor: Manzoni, Bruno, 24030 Berbenno, (Bergamo) (IT)
(74) Representative: Luksch, Giorgio, Dr.-Ing.

(57) **Abstract**

This phyto-derivative disinfectant solution for veterinary use in the endo-mammary anti-mastitis treatment of milk-producing animals has the characteristic of associating anti-inflammatory properties deriving from vegetable extracts of Melaleuca Alternifolia, Achinacea Angustifolia, Calendula officinalis and Propolis dissolved in water by known methods in the following percentages:
0.2%-2.5% Essential oil obtained by distilling the foliage of Melaleuca Alternifolia in a current of steam and having a minimum TERPINEN-4-OL content of 35% and a maximum 1,8 CINEOLE content of 5%;
0.5%-1.5% ACHINACEA ANGUSTIFOLIA extract;
0.5%-1.5% PROPOLIS extract;
0.5%-1.5% CALENDULA OFFICINALIS extract;
said extracts being dissolved in that quantity of water required to achieve 100 and being associated with gelling agents, preferably CARBOMER, in a percentage from 0.1 to 0.5%.

## Description

This invention relates to a phyto-derivative disinfectant solution for veterinary use in the endo-mammary anti-mastitis treatment of milk-producing animals.

As is well known, mastitis represents a serious recurring problem in the industrial production of milk. Its resultant mammary inflammations are generally divided into "contagious" mastitis and "environmental" mastitis. The former are sustained by germs of endo-mammary location, in particular Staph., Agalactiae and Staph. Aureus. The said endo-mammary located pathogenic agents are currently combated with bactericides of various types: for example chlorhexidine digluconate or chlorine dioxide. The currently used bactericides often have their active principles based on the action of antibiotics with undesirable side effects, which are not always known as they appear only after very lengthy time periods and as pathologies which only by considerable experimentation (not always economically possible) could be proved to have derived from the use of those antibiotics. In this respect, the considerable veterinary attention given to milk-producing animals (particularly cows) makes it very probable that they have also been given other antibiotics, which could determine dangerous anaphylactic reactions. In any event, considering that said antibiotic substances are in fact in circulation within the organism of the milk-producing cattle concerned, the possibility of transfer of these dangerous substances into the milk produced cannot be discounted.

From the aforegoing it is clear that the results, which derive from the general principles of the said anti-mastitis products of allopathic conception, are results having their own specificity: namely in terms of overall treatment costs, in terms of health of the udder and in terms of hygiene of the milk produced by animals under pharmacological treatment.

To obviate these serious intrinsic problems of the usual antibiotic drugs (or allopathic disinfectants), homeopathic therapies have been attempted. In this respect, the use of homeopathic substances certainly leaves no toxic residues in the animal organism or in the milk produced by it, but the fact remains that homeopathy is based on doubtful concepts not acceptable to modern scientific thought, making any success obtained problematical.

Although the complexity of homeopathic diagnosis leads to a clinical symptom framework of such vast complexity as to be able to attribute lack of therapeutic success to the lack of skill of the homeopath, the fact remains that homeopathy can be relegated to the level of pure credulity.

An object of the present invention is to define a pharmacological product of allopathic conception which is suitable for endo-mammary use. A further object is to define a product, as aforestated, which does not create complications for the animal's health. A further object is to define a product which enables the milk to be used even if milked immediately after treatment. These and further objects will be seen to have been attained from the ensuing description of a phyto-derivative disinfectant solution for veterinary use in the endo-mammary anti-mastitis treatment of milk-producing animals having the characteristic of associating anti-inflammatory properties deriving from vegetable extracts of Melaleuca Alternifolia, Achinacea Angustifolia, Calendula officinalis and Propolis dissolved in water by known methods in the following percentages:
0.2%-2.5% Essential oil obtained by distilling the foliage of Melaleuca Alternifolia in a current of steam and having a minimum TERPINEN-4-OL content of 35% and a maximum 1,8 CINEOLE content of 5%;
0.5%-1.5% ACHINACEA ANGUSTIFOLIA extract;
0.5%-1.5% PROPOLIS extract;
0.5%-1.5% CALENDULA OFFICINALIS extract;
said extracts being dissolved in that quantity of water required to achieve 100 and being associated with gelling agents, preferably Carbomer, in a percentage from 0.1 to 0.5%.

The aforesaid Melaleuca oil is extracted by distilling the foliage of Melaleuca Alternifolia in a current of steam. It is composed of a mixture of monoterpenes, sesquiterpenes and terpinic alcohols in which the TERPINEN-4-OL content is not less than 30% in order to exert the best germicidal action and the 1,8 CINEOLE content does not exceed 15% in order not to generate mucosa irritation. The maximum percentage of TERPINEN-4-OL beyond which bactericidal power does not increase is 40%. Other important components present in said oil (there are more than 90) include:
α-terpineol, in a quantity variable from 1.5 to 8%;
τ-terpinene, in a quantity variable from 10 to 28%;
α-terpinene, in a quantity variable from 5 to 13%;
p-cymene, in a quantity variable from 0.5 to 12%.

This oil is present in the solution of the invention in a percentage variable from 0.2 to 2.5%, depending on formulations aimed at more or less rapid absorption. The totality of said products of the formulation is complemented by that percentage of water required to achieve 100. This water is demineralized by reverse osmosis and prefiltration to 1 micron; the water is also sterilized by suitable ultraviolet lamps.

With regard to the other active principles of the solution, the ACHINACEA ANGUSTIFOLIA extract performs a local anti-bacterial and anti-inflammatory action on the udder and a detoxifying action on the organism.

The CALENDULA OFFICINALIS extract performs a protective anti-inflammatory action on the mammary parenchyma, and also a mild bacteriostatic action.

The PROPOLIS extract is also a product possessing anti-bacterial and anti-inflammatory action; it is particularly able to exert a synergic action with the other anti-bacterial agents.

To stabilize the solution a gelling agent is used, preferably of the type known commercially as CARBOMER.

## Claims

1. A phyto-derivative disinfectant solution for veterinary use in the endo-mammary anti-mastitis treatment of milk-producing animals, **characterised by** associating therapeutic and anti-inflammatory properties deriving from vegetable extracts of Melaleuca Alternifolia, Achinacea Angustifolia, Calendula officinalis and Propolis dissolved in water by known methods in the following percentages:
0.2%-2.5% Essential oil obtained by distilling the foliage of Melaleuca Alternifolia in a current of steam and having a minimum TERPINEN-4-OL content of 35% and a maximum 1,8 CINEOLE content of 5%;
0.5%-1.5% ACHINACEA ANGUSTIFOLIA extract;
0.5%-1.5% PROPOLIS extract;
0.5%-1.5% CALENDULA OFFICINALIS extract;
said extracts being dissolved in that quantity of water required to achieve 100 and being associated with gelling agents, preferably CARBOMER, in a percentage from 0.1 to 0.5%.
